# EUROPÄISCHE PATENTSCHRIFT

(11) **EP 0 479 043 B1**
(45) Veröffentlichungstag und Bekanntmachung des Hinweises auf die Patenterteilung: **08.11.1995**
(21) Anmeldenummer: 91115811.1
(22) Anmeldetag: 18.09.1991
(51) Int. Cl.: C12P 7/62

(54) **Verfahren zur Gewinnung eines Polyhydroxyalkanoats aus dem Zell-material eines Mikroorganismus**
Process for the production of a polyhydroxyalkanoate from the cell material of a microorganism
Procédé de production d'un polyhydroxyalcanoate à partir du matériel cellulaire d'un micro-organisme

(30) Priorität: 05.10.1990 AT 2018/90
(43) Veröffentlichungstag der Anmeldung: 08.04.1992
(73) Patentinhaber: PCD-Polymere Gesellschaft m.b.H., A-2323 Schwechat-Mannswörth (AT)
(72) Erfinder: Blauhut, Wilfried, Dipl.-Ing., A-4040 Linz (AT); Gierlinger, Wolgang, A-4030 Linz (AT); Strempel, Friedrich, Dipl.-Ing.Dr., A-4020 Linz (AT)
(74) Vertreter: Kunz, Ekkehard, Dr.

(56) Entgegenhaltungen:
- EP-A- 0 014 490
- EP-A- 0 036 699
- EP-A- 0 046 017
- EP-A- 0 124 309
- EP-A- 0 168 095
- EP-A- 0 288 908
- EP-A- 0 304 293
- EP-A- 0 355 307
- A. FRANCK et al., "Kunststoff-Kompendium", 2. Auflage, Vogel-Verlag, Würzburg (DE), 1988; p. 234/

## Beschreibung

Die Erfindung betrifft ein Verfahren zur Gewinnung eines durch einen Mikroorganismus intrazellulär aufgebauten Polyhydroxyalkanoates aus dem Zellmaterial.

Polyhydroxyalkanoate, insbesondere Homo- und Copolymere der D-(-)3-Hydroxybuttersäure (Poly-HB) werden als Speicherstoff für Energie und Kohlenstoff von vielen Mikroorganismen intrazellulär aufgebaut und angereichert und stellen Polyester mit thermoplastischen Eigenschaften dar, die biologisch abbaubar sind. Poly-HB kann mit Hilfe von Mikroorganismen beispielsweise nach der in EP-A-0 144 017 oder EP-A-0 149 744 beschriebenen Verfahrensweise in guten Ausbeuten hergestellt werden. Copolyester von Poly-HB, wie z.B. Copolyester, die aus 3-Hydroxybuttersäure- und 3-Hydroxyvaleriansäureeinheiten oder auch anderen Säureeinheiten bestehen, können beispielsweise nach einer der in EP-A-0 052 459, EP-A-0 204 442, EP-A-0 288 908, EP-A-0 304 293 oder EP-A-0 274 151 beschriebenen Vorgangsweise auf fermentativem Weg hergestellt werden. Die gebildeten Polyhydroxyalkanoate sind im Zellmaterial des Mikroorganismus eingeschlossen und müssen vom Zellmaterial getrennt werden, was erhebliche Schwierigkeiten bereitet. Eine der Möglichkeiten zur Abtrennung ist die Extraktion mit Hilfe eines Lösungsmittels, wobei die Durchführung der bisher beschriebenen Verfahren ebenfalls mit Schwierigkeiten verbunden ist.

Um nämlich das Polyhydroxyalkanoat, das im Zellmaterial des Mikroorganismus eingeschlossen ist, dem Angriff des Lösungsmittels besser zugänglich zu machen, mußte dem eigentlichen Extraktionsschritt ein gesonderter Schritt zum Aufbrechen oder Löslichmachen des Zellmaterials vorgeschaltet werden. So ist beispielsweise in US-A-3.044.942 eine Vorbehandlung der Zellen mit Aceton, in US-A-3.275.610 eine mechanische Vorbehandlung der Zellen durch Zerstampfen der Zellen oder Schütteln mit harten Gegenständen, in EP-A-0 015 123, EP-A-0 124 309 oder EP-A-0 168 095 eine Sprühtrocknung oder andere Vortrocknung der Mikroorganismenzellen, beispielsweise durch azeotropes Abdestillieren des Wassers und in EP-A-0 015 669 eine Vorbehandlung der Zellen durch osmotischen Schock, Ultraschall oder Zellwandlyse beschrieben. Weiters bereitet die Abtrennung des so vorbehandelten ungelösten Zellmaterials von dem gelösten Polyhydroxyalkanoat große Schwierigkeiten, da das ungelöste Zellmaterial als gallertartige Masse die Filter verstopft und die Abtrennung der organischen Phase vom Zellmaterial kaum vollständig durchführbar ist. Aus diesem Grund wird in EP-A-0 046 017 beschrieben, das Zellmaterial vor der Extraktion mit Hilfe einer kombinierten Alkali-, Säure- und Hitzebehandlung zwecks besserer Abtrennbarkeit auszuflocken, während in CA Vol.108, 1988, Ref.73835j der Einsatz einer Filterhilfe wie Perlit oder Diatomeenerde zur Abtrennung des Zellmaterials vom Lösungsmittel vorgeschlagen wird.

Aus EP-A-0 036 699 und EP-A 0 014 490 ist ein Verfahren zur Gewinnung von Poly-HB bekannt, bei dem ein Teil des Wassers aus der Zellsuspension entfernt und das Zellmaterial mit einem mit Wasser nicht mischbaren organischen Lösungsmittel für das Poly-HB bei 10 bis >50° C gerührt wird. Das Lösungsmittel weist einen Siedepunkt von weniger als 100° C auf. Nach Abtrennen der organischen Phase wird das Poly-HB durch Zugabe eines Non-Solvens ausgefällt und nach Verdampfen des Lösungsmittels gewonnen.
Weiters ist unter anderem im Kunststoff-Kompendium, Franck-Biederbick, 2. Aufl., Würzburg, Vogel-Verlag 1988, Seite 234 die Herstellung von Zellwolle nach dem Viskoseverfahren beschrieben, nach welchem Cellulose mit CS₂ chemisch zu Cellulosexanthogenat umgesetzt und anschließend durch ein schwefelsaures Fällbad gepreßt wird, wobei die Fäden mechanisch verstreckt werden. Die Cellulose bildet sich zurück und CS₂ entweicht.

Es wurde nun unerwarteterweise ein einfaches Verfahren gefunden, bei dem die Zellen nicht aufgebrochen werden müssen und bei dem die Zellrückstände ohne jeglichen Aufwand und ohne Schwierigkeiten entfernt werden, wobei gleichzeitig das Polyhydroxyalkanoat in Form gut handzuhabender Flocken isoliert wird, die überraschend gut zu Gegenständen weiterverarbeitbar sind.

Gegenstand der Erfindung ist demnach ein Verfahren zur Gewinnung eines durch einen Mikroorganismus intrazellulär aufgebauten Polyhydroxyalkanoates aus dem Zellmaterial einer fermentierten, wäßrigen Zellsuspension, bei dem zumindest ein Teil der Fermentierlösung oder des in der Fermentierlösung enthaltenen Wassers aus der Zellsuspension entfernt wird, worauf das Zellmaterial mit einem organischen Lösungsmittel für das Polyhydroxyalkanoat, das mit Wasser nicht mischbar ist und das einen Siedepunkt von weniger als 100 °C aufweist mit oder ohne Zusatz von Wasser, versetzt wird, worauf die entstandene Extraktionsmischung bei Temperaturen von Raumtemperatur bis zum Siedepunkt des organischen Lösungsmittels gerührt, mit oder ohne Zentrifugieren unter Ausbildung einer wäßrigen und einer organischen Phase absetzen gelassen und die organische Phase, die das Polyhydroxyalkanoat gelöst enthält von der wäßrigen Phase, die die Zellrückstände ungelöst enthält, abgetrennt wird, das dadurch gekennzeichnet ist, daß die organische Phase in heißes Wasser einer Temperatur, die höher ist als der Siedepunkt des organischen Lösungsmittels, eingedüst wird, wodurch das organische Lösungsmittel verdampft, das Polyhydroxyalkanoat im Wasser ausfällt und auf übliche Art und Weise gewonnen wird.

Zur Durchführung des erfindungsgemäßen Verfahrens wird zuerst aus der fermentierten, wäßrigen Zellsuspension zumindestens ein Teil der Fermentierlösung oder des Wassers entfernt. Dazu können Trennverfahren wie z.B. Abdekantieren, Abzentrifugieren, Abfilterien des Zellmaterials von der Fermentierlösung eingesetzt werden, oder es wird zumindestens ein Teil des in der Fermentierlösung enthaltenen Wassers durch Abdestillieren entfernt. Bevorzugt wird ein Teil der Fermentierlösung durch Abzentrifugieren vom Zellmaterial, bevorzugt mit Hilfe eines Separators entfernt. Dadurch wird die Konzentration der Suspension an Zellen erhöht, was für das weitere Verfahren von Bedeutung ist.

Einer der Vorteile des erfindungsgemäßen Verfahrens liegt darin, daß das Zellmaterial nicht durch Aufbrechen oder Trocknen vorbehandelt werden muß. Es ist aber auch möglich, vorbehandeltes Zellmaterial im erfindungsgemäßen Verfahren einzusetzen.

Das vorzugsweise Wasser enthaltende Zellmaterial oder die konzentrierte Zellsuspension wird mit einem organischen Lösungsmittel für das Polyhydroxyalkanoat, das mit Wasser nicht mischbar ist und das einen Siedepunkt aufweist, der unter dem des Wassers liegt, versetzt. Geeignete Lösungsmittel sind etwa halogenierte Kohlenwasserstoffe wie Methylenchlorid, Chloroform, Trichlorethylen. Bevorzugt wird Methylenchlorid eingesetzt.
Gegebenenfalls wird der Mischung aus Zellmaterial und organischem Lösungsmittel auch noch Wasser zugesetzt, da es wesentlich ist, daß ein optimales Verhältnis von Zellmaterial, organischem Lösungsmittel und Wasser entsteht, damit später eine optimale Phasentrennung erfolgt.

Bevorzugt wird soviel organisches Lösungsmittel und soviel Wasser zugegeben, daß nach der Zugabe das Gewichtsverhältnis Zellmaterial bezogen auf das Zelltrockengewicht : Wasser : Lösungsmittel etwa 2 : 1 : 10 bis 1 : 10 : 100, besonders bevorzugt etwa 2 : 3 : 20 bis 1 : 5 : 50, ganz besonders bevorzugt etwa 1 : 3 : 20, beträgt.

Die Mischung aus Zellmaterial, organischem Lösungsmittel für das Polyhydroxyalkanoat und Wasser wird bei Temperaturen von Raumtemperatur bis zum Siedepunkt des verwendeten Lösungsmittels gut gerührt. Bevorzugt wird die Mischung mit Hilfe von Mischern, die ein intensives Durchmischen der Phasen ermöglichen, beispielsweise mit Hilfe von Statikmischern oder dynamischen Intensivmischern, gerührt, um das Polyhydroxyalkanoat möglichst vollständig aus dem Zellmaterial herauszulösen. Bei Verwendung von Intensivmischern ist ein Erhitzen des Lösungsmittels nicht nötig, da schon bei Raumtemperatur das Polyhydroxyalkanoat praktisch vollständig aus dem Zellmaterial herausgelöst wird. Nach dem intensiven Durchmischen und gegebenenfalls nach dem Abkühlen wird das Gemisch absitzen gelassen oder es wird zentrifugiert, wobei sich eine wäßrige und eine organische Phase ausbilden. Bevorzugt wird das Gemisch zentrifugiert, da die Phasentrennung auf diese Weise erleichtert wird. Die wäßrige Phase enthält danach den unlöslichen Zellrückstand, die organische Phase das gelöste Polyhydroxyalkanoat. Zur Entfernung des Zellmaterials ist keine Filtration nötig, da die organische Phase durch einfache Phasentrennung von der wäßrigen Phase abgetrennt wird, wobei die Zellrückstände in der wäßrigen Phase verbleiben.
Es kann vorkommen, daß die organische Lösung trüb anfällt.

Zur Entfernung der Trübung kann die organische Lösung durch ein Tiefenfilter, z.B. durch ein Sandbett durchgeleitet werden.

Die abgetrennte, organische Phase, welche das Polyhydroxyalkanoat gelöst enthält, wird anschließend in heißes Wasser eingedüst, das in einem Behälter vorgelegt wird.
Die Temperatur des vorgelegten Wassers muß über dem Siedepunkt des organischen Lösungsmittels und unter dem Siedepunkt des Wassers, das auch unter Druck vorgelegt werden kann, liegen. Zum Eindüsen der organischen Phase können alle geeigneten Düsen, wie Einstoff- oder Zweistoffdüsen geeigneter Bauart verwendet werden. Vorteilhafterweise wird die organische Phase mit Hilfe von Wasserdampf als Treibmittel mittels einer Zweistoffdüse in das heiße Wasser eingedüst. Sobald das organische Lösungsmittel mit dem vorgelegten, heißen Wasser in Berührung kommt, verdampft das organische Lösungsmittel und das Polyhydroxyalkanoat fällt im vorgelegten Wasser in Form von Flocken aus.

Da sich das vorgelegte heiße Wasser durch das Verdampfen des organischen Lösungsmittels abkühlt, wird der Wasserbehälter vorteilhafterweise etwa mit Hilfe einer Mantelheizung beheizt und auf annähernd konstanter Temperatur gehalten, wobei bei der Verwendung von Wasserdampf als Treibmittel zumindest ein Teil der Verdampfungswärme des Lösungsmittels durch die Kondensationswärme des Treibdampfes aufgebracht wird.

Die Menge des vorgelegten, heißen Wassers ist für den Erfolg der Verfahrensdurchführung nicht ausschlaggebend. Vorteilhafterweise wird aber soviel Wasser vorgelegt, daß durch einfaches Rühren des Vorlageninhaltes ein Absetzen des abgeschiedenen Polyhydroxyalkanoates vermieden wird. Dies ist bei einer Polyhydroxyalkanoatkonzentration von etwa 3 % im allgemeinen noch der Fall.
Das verdampfte organische Lösungsmittel und der entsprechend den Dampfdruckverhältnissen mitgeführte Wasserdampf werden außerhalb des Wasserbehälters auf übliche Weise kondensiert. Das kondensierte Wasser kann unmittelbar in den Wasserbehälter zurückgeführt und das organische Lösungsmittel erneut in ein Verfahren nach Anspruch 1 eingesetzt werden.

Die ausgefallenen Polyhydroxyalkanoatflocken werden anschließend im heißen Wasser 15 bis 60, bevorzugt 20 bis 40 Minuten gerührt, um das organische Lösungsmittel möglichst vollständig auszutreiben und anschließend durch geeignete Methoden, wie Filtrieren, Absaugen, Abzentrifugieren, bevorzugt durch Abzentrifugieren vom Wasser abgetrennt und auf geeignete Weise, beispielsweise durch Trocknung im Vakuum, Trocknung in Hordenöfen, bei Temperaturen von 60 bis 110 °C getrocknet.

Die dabei entstandenen Flocken aus Polyhydroxyalkanoat sind hervorragend handzuhaben, da diese Flocken praktisch keinen Staubanteil enthalten und als Haufwerk sehr gut durchströmbar sind.
Das Verfahren kann kontinuierlich oder diskontinuierlich durchgeführt werden, wird aber bevorzugt kontinuierlich durchgeführt.

Nach einer bevorzugten Ausführungsform wird das Poly-HB enthaltende Zellmaterial von der Fermentierlösung abzentrifugiert, worauf dem Zellmaterial soviel Methylenchlorid und gegebenenfalls soviel Wasser zugesetzt wird, daß das Gewichtsverhältnis von Zellmaterial bezogen auf das Zelltrockengewicht : Wasser : Methylenchlorid etwa 1 : 3 : 10 bis 1 : 5 : 30 beträgt. Die entstandene Mischung wird anschließend entweder unter Rühren 10 bis 60 Minuten auf Rückfluß erhitzt oder bei Raumtemperatur mit Hilfe eines dynamischen Intensivmischers gerührt, gegebenenfalls abgekühlt und zentrifugiert, wobei sich eine wäßrige Phase, die die Zellrückstände ungelöst und eine organische Phase, die die Poly-HB gelöst enthält, ausbildet. Die Methylenchloridphase wird von der wäßrigen Phase abgelassen und mit Hilfe von Wasserdampf in einen geheizten Behälter, in dem Wasser einer Temperatur von 70 bis 90 °C gerührt vorgelegt wurde, eingedüst. Die dadurch im Wasser in Flocken ausgefällte Poly-HB wird 20 bis 40 Minuten im heißen Wasser gerührt, mit Hilfe einer Zentrifuge abgetrennt und in einem Hordenofen bei 80 bis 100 °C getrocknet.

Die Polyhydroxyalkanoate, die nach dem erfindungsgemäßen Verfahren erhalten werden, lassen sich unerwartet gut zu Gegenständen weiterverarbeiten. So werden beispielsweise beim Spritzgußverfahren annähernd gleiche Zykluszeiten, wie bei Polypropylen erreicht. Dies ist für den Fachmann völlig überraschend, da bekannt ist, daß Polyhydroxyalkanoate langsamer kristallisieren als Polypropylen.

Auf die beschriebene Art und Weise wird das Polyhydroxyalkanoat in guten Ausbeuten auf einfache Weise vom Zellmaterial des Mikroorganismus abgetrennt, wobei das Polyhydroxyalkanoat in Form von Flocken anfällt, die gut handzuhaben und gut weiterzuverarbeiten sind. Das Verfahren stellt somit eine Bereicherung der Technik dar.

### Beispiel 1

60 l einer wäßrigen Fermentierlösung, die 26 Gew.% eines Zellmaterials von Alcaligenes latus mit einem Poly-HB Gehalt von 72 Gew.% enthielt, erhalten nach der in EP-A-0 144 017 beschriebenen Vorgangsweise und Abzentrifugieren eines Teils der Fermentierlösung mittels eines Tellerseparators mit Feststoffaustrag, wurde mit 30 l Wasser und 400 l Methylenchlorid versetzt und unter Rühren 30 Minuten auf Rückfluß erhitzt. Das entstandene Gemisch wurde in einer Siphonzentrifuge mit einem Trommeldurchmesser von 630 mm bei 2200 U/min zentrifugiert, wobei sich eine wäßrige Phase, die das Mikroorganismenzellmaterial ungelöst und eine organische Phase, die Poly-HB gelöst enthielt, bildete. Die organische Unterphase wurde von der wäßrigen Oberphase abgelassen und mittels einer Zweistoffdüse mit einer Bohrung von 4 mm Durchmesser für die PHB-Lösung und ca. 2 mm Ringspaltweite für das Treibmittel, nämlich Wasserdampf, mit einem Treibmittelvordruck von 3 bar in 800 l Wasser einer Temperatur von 80 °C, das in einem gerührten, Behälter vorgelegt worden war, mit einem Volumenstrom von 300 l/h eingedüst. Die Temperatur des Wassers wurde dabei mit Hilfe einer Mantelheizung, die den Behälter umgab, annähernd konstant gehalten. Dabei fiel Poly-HB in Form von Flocken aus, während das Methylenchlorid und ein kleiner Teil des Wassers verdampfte und außerhalb des Behälters kondensiert und aufgefangen wurde. Nach Beendigung des Eindüsens wurde die Suspension 30 Minuten bei 80 °C gerührt. Anschließend wurde der Behälterinhalt in eine Schälzentrifuge mit einem Trommeldurchmesser von 630 mm gepumpt und bei einer Schleuderdrehzahl von 2000 U/min in Wasser und Zentrifugenfeuchte Poly-HB-Flocken getrennt. Die zentrifugenfeuchten Flocken wurden in einem Hordentrockner bei 80 °C 24 Stunden getrocknet.

Dabei wurden 9,5 kg Poly-HB, das sind 85 % der Theorie mit einer Reinheit >99 %, und einem Gehalt an Methylenchlorid von <1 ppm erhalten.

### Beispiel 2

50 ml einer wäßrigen, wie im Beispiel 1 beschriebenen, Fermentierlösung, wurden mit 20 ml Wasser und 350 ml Methylenchlorid versetzt und bei Raumtemperatur mit Hilfe eines Ultra Turrax Intensivmischers der Firma IKA, Maschinenbau, Janke & Kunkel GmbH, Deutschland, 2 Minuten gerührt. Dabei gingen 97 Gew.% der im Zellmaterial vorhanden gewesenen Poly-HB in Lösung.

## Patentansprüche

1. Verfahren zur Gewinnung eines durch einen Mikroorganismus intrazellulär aufgebauten Polyhydroxyalkanoates aus dem Zellmaterial einer fermentierten, wäßrigen Zellsuspension, bei dem zumindest ein Teil der Fermentierlösung oder des in der Fermentierlösung enthaltenen Wassers aus der Zellsuspension entfernt wird, worauf das Zellmaterial mit einem organischen Lösungsmittel für das Polyhydroxyalkanoat, das mit Wasser nicht mischbar ist und das einen Siedepunkt von weniger als 100 °C aufweist mit oder ohne Zusatz von Wasser, versetzt wird, worauf die entstandene Extraktionsmischung bei Temperaturen von Raumtemperatur bis zum Siedepunkt des organischen Lösungsmittels gerührt, mit oder ohne Zentrifugieren unter Ausbildung einer wäßrigen und einer organischen Phase absetzen gelassen und die organische Phase, die das Polyhydroxyalkanoat gelöst enthält von der wäßrigen Phase, die die Zellrückstände ungelöst enthält, abgetrennt wird, dadurch gekennzeichnet, daß die organische Phase in heißes Wasser einer Temperatur, die höher ist als der Siedepunkt des organischen Lösungsmittels, eingedüst wird, wodurch das organische Lösungsmittel verdampft, das Polyhydroxyalkanoat im Wasser ausfällt und auf übliche Art und Weise gewonnen wird.

2. Verfahren nach Anspruch 1, dadurch gekennzeichnet, daß das Polyhydroxyalkanoat ein Homo- oder Copolymer der Poly-D(-)-3-hydroxybuttersäure ist.

3. Verfahren nach einem der Ansprüche 1 oder 2, dadurch gekennzeichnet, daß zumindest ein Teil der Fermentierlösung durch Zentrifugieren aus der Zellsuspension entfernt wird.

4. Verfahren nach einem der Ansprüche 1 bis 3, dadurch gekennzeichnet, daß das Zellmaterial mit soviel organischem Lösungsmittel und soviel Wasser versetzt wird, daß das Gewichtsverhältnis von Zellmaterial bezogen auf das Zelltrockengewicht : Wasser : Lösungsmittel von 2 : 3 : 20 bis 1 : 5 : 50 beträgt.

5. Verfahren nach einem der Ansprüche 1 bis 4, dadurch gekennzeichnet, daß die Extraktionsmischung nach dem Rühren zentrifugiert wird.

6. Verfahren nach einem der Ansprüche 1 bis 5, dadurch gekennzeichnet, daß die organische Phase mit Hilfe von Wasserdampf als Treibmittel in heißes Wasser eingedüst wird.

7. Verfahren nach einem der Ansprüche 1 bis 6, dadurch gekennzeichnet, daß das ausgefällte Polyhydroxyalkanoat 20 bis 40 Minuten im heißen Wasser gerührt wird.

## Claims

1. Process for obtaining a polyhydroxyalkanoate, synthesised intracellularly by a microorganism, from the cell material of a fermented, aqueous cell suspension, in which process at least part of the fermentation solution, or of the water contained in the fermentation solution, is removed from the cell suspension, after which the cell material is treated with an organic solvent for the polyhydroxyalkanoate, which is immiscible with water and which has a boiling point of less than 100°C, with or without the addition of water, whereupon the extraction mixture formed is stirred at temperatures from room temperature to the boiling point of the organic solvent and is allowed to settle, with or without centrifugation, forming an aqueous and an organic phase, and the organic phase which contains the polyhydroxyalkanoate in dissolved form is separated from the aqueous phase which contains the cell residues in undissolved form, which process is characterised in that the organic phase is injected into hot water at a temperature which is higher than the boiling point of the organic solvent, causing the organic solvent to evaporate and the polyhydroxyalkanoate to precipitate in the water, the latter being obtained in the customary fashion.

2. Process according to Claim 1, characterised in that the polyhydroxyalkanoate is a homopolymer or copolymer of poly-D(-)-3-hydroxybutyric acid.

3. Process according to one of Claims 1 and 2, characterised in that at least part of the fermentation solution is removed from the cell suspension by centrifugation.

4. Process according to one of Claims 1 to 3, characterised in that the cell material is treated with such an amount of organic solvent and such an amount of water that the ratio by weight of cell material relative to the cell dry weight : water : solvent is from 2 : 3 : 20 to 1 : 5 : 50.

5. Process according to one of Claims 1 to 4, characterised in that the extraction mixture is centrifuged after stirring.

6. Process according to one of Claims 1 to 5, characterised in that the organic phase is injected into hot water with the aid of steam as the propellant.

7. Process according to one of Claims 1 to 6, characterised in that the precipitated polyhydroxyalkanoate is stirred in the hot water for 20 to 40 minutes.

## Revendications

1. Procédé de préparation d'un polyhydroxyalcanoate formé de façon intracellulaire par un micro-organisme, à partir du matériel cellulaire d'une suspension cellulaire aqueuse fermentée, dans lequel au moins une partie de la solution de fermentation ou de l'eau contenue dans la solution de fermentation est chassée de la suspension cellulaire, après quoi on ajoute au matériau cellulaire un solvant organique pour le polyhydroxyalcanoate, qui n'est pas miscible avec l'eau et présente un point d'ébullition situé au-dessous de 100°C, avec ou sans addition d'eau, ensuite, on agite le mélange d'extraction formé, à des températures comprises entre la température ambiante et le point d'ébullition du solvant organique, on le laisse reposer, avec ou sans centrifugation, on obtient la formation d'une phase aqueuse et d'une phase organique, et on sépare la phase organique, qui contient le polyhydroxyalcanoate à l'état dissous, de la phase aqueuse qui contient les résidus cellulaires non dissous, caractérisé en ce qu'on introduit la phase organique, par une tuyère, dans de l'eau chaude, à une température supérieure au point d'ébullition du solvant organique, grâce à quoi le solvant organique est évaporé, le polyhydroxyalcanoate précipite dans l'eau, et on le recueille d'une manière usuelle.

2. Procédé selon la revendication 1, caractérisé en ce que le polyhydroxyalcanoate est un homopolymère ou un copolymère de l'acide poly-D-(-)-3-hydroxybutyrique.

3. Procédé selon la revendication 1 ou 2, caractérisé en ce qu'au moins une partie de la solution de fermentation est chassée de la suspension cellulaire par centrifugation.

4. Procédé selon l'une des revendications 1 à 3, caractérisé en ce qu'on ajoute au matériel cellulaire des quantités de solvant organique et d'eau telles que le rapport pondéral du matériel cellulaire exprimé en poids net de cellules : eau : solvant est compris entre 2 : 3 : 20 et 1 : 5 : 50.

5. Procédé selon l'une des revendications 1 à 4, caractérisé en ce qu'on centrifuge le mélange d'extraction après l'avoir agité.

6. Procédé selon l'une des revendications 1 à 5, caractérisé en ce qu'on introduit la phase organique, par une tuyère, dans de l'eau chaude à l'aide de vapeur d'eau comme propulseur.

7. Procédé selon l'une des revendications 1 à 6, caractérisé en ce qu'on agite le polyhydroxyalcanoate précipité dans de l'eau chaude pendant 20 à 40 minutes.
